# EUROPEAN PATENT APPLICATION

(11) **EP 4 729 902 A1**
(43) Date of publication of application: **22.04.2026**
(21) Application number: 24207408.6
(22) Date of filing: 18.10.2024
(51) Int. Cl.: G01K 1/10, G01K 13/02

(54) **THERMOMETER WITH GAS SENSOR**

(71) Applicant: Endress+Hauser Wetzer GmbH+Co. KG, 87484 Nesselwang (DE)
(72) Inventor: Fortunato, Gianluca, Milano 20153 (IT)
(74) Representative: Endress + Hauser Group Services (Deutschland) AG+Co. KG

(57) **Abstract**

The invention relates to an apparatus (100) for determining and/or monitoring the temperature (T) of a medium (M) in container (10), with a temperature sensor (1) and a gas sensor (2) arranged in an interior of an elongated tubular member (4) serving as a sheath. The invention further relates to a method for operating such an apparatus (100).

## Description

The invention relates to an apparatus for determining and/or monitoring the temperature of a medium with a gas sensor.

Thermometers are known from the prior art in a great variety of embodiments. Thus, there are thermometers which use the expansion of a liquid, a gas or a solid with a known coefficient of expansion, in order to measure temperature, or also others which relate the electrical conductivity of a material, or a quantity derived therefrom, to the temperature, such as electrical resistance when using, for example, resistance elements, or the thermoelectric effect in the case of thermocouples. On the other hand, radiation thermometers, in particular pyrometers, use the heat radiation of a substance to determine its temperature. The underlying measurement principles have each been described in a variety of publications.

In the case of a temperature sensor in the form of a resistance element, so-called thermistors have become known. They are also referred to as PTC or NTC thermistors - short for positive or negative temperature coefficient, respectively. PTC thermistors are particularly suitable as temperature sensors since their resistance increases linearly to first order as the temperature rises. Among the PTC thermistors, platinum is again a preferred material and widely used, since platinum exhibits an overall dependence of resistance on temperature that is at most quadratic. Resistance elements are typically designed to have a certain nominal resistance at a reference temperature of 0°C and are commercially available as so-called Pt10 (10 Ohm), Pt100 (100 Ohm) and Pt1000 (1 kOhm) resistance elements.

The resistance element may be embodied as a so-called wound resistance thermometer or is embodied as a layer mounted on a substrate in thin- or thick film technology. In the case of a thin-film sensor, for example, a sensor element provided with connecting wires and mounted on a carrier substrate is used, wherein the back side of the carrier substrate usually has a metal coating.

In the case of temperature sensors in the form of thermocouples, however, the temperature is determined by a thermovoltage which arises between the unilaterally connected thermowires made of different materials. Thermocouples according to standard IEC 60584-x, e.g., thermocouples of the types R, S, B, J, T, E, K, N, C and A, are usually used as temperature sensors for temperature measurement.

However, other pairs of materials, for example those with a measurable Seebeck effect, are also possible.

Often, the temperature sensor is arranged in a so-called measuring insert, comprising an elongated tubular member serving as a sheath for the temperature sensor. The sheath contains e.g. an electrically isolating filler into which the temperature sensor, and at least part of electrical connection lines, which serve for contacting the temperature sensor, are embedded. Typically, for example for an insertion thermometer, the measuring insert protrudes into the medium for the determination of the temperature, starting from a distal end region of the measuring insert. Thus, the distal end region faces the medium and/or dips into the medium and the proximal end region (which is opposite the distal end region in the longitudinal direction of the measuring insert) faces away from the medium. The elongated tubular member is especially closed or sealed off at a distal end region.

The measuring insert may be assembled in a protective tube, especially a metallic protective tube, for example a thermowell. Also, the protective tube is typically sealed off at its distal end, to protect the components, for example the measuring insert and/or the temperature sensor, against an environment to which the thermometer is exposed, e.g., a medium into which the thermometer is inserted. Typically, the measuring insert is, especially repeatedly, removable from the protective tube, for the purpose of the maintenance and/or replacement or the measuring insert.

Thermometers are increasingly used for determining the temperature of a medium in a container, comprising at least a gaseous component, or a completely gaseous medium stored in a gas-tight closed and/or pressurized container. For a medium comprising gaseous or liquified H2, a process known as hydrogen embrittlement may occur, caused by gaseous hydrogen penetrating the material and producing chemical-mechanical damage to the measuring insert. This weakens the mechanical stability and/or gas tightness of the measuring insert. In a worst-case scenario, gas may diffuse from the container through the measuring insert into the environment. It is therefore important to detect a leakage of at least one target gas to be detected into the thermometer before the gas can escape to the environment of the thermometer.

The European Patent application EP 3 118 583 A1 discloses a thermometer with a gas sensor as a sensing element of a failure detection unit. The failure detection unit with the gas sensor is part of a modular sealing apparatus. However, the gas sensor is arranged outside the measuring insert. Thus, the gas sensor is not able to measure a gas concentration inside the measuring insert, in the vicinity of the temperature sensor.

It is therefore the object of the present invention to provide a thermometer which can accurately determine the gas concentration in the measuring insert.

The object is achieved by an apparatus for determining and/or monitoring the temperature of a medium and a method of operating an apparatus according to the invention. Advantageous embodiments are respectively specified in the dependent claims, wherein all embodiments of the apparatus according to the invention are, of course, mutatis mutandis encompassed by the method according to the invention and vice versa.

Regarding the apparatus, the object underlying the invention is achieved by an apparatus for determining and/or monitoring the temperature of a medium, the apparatus comprising:
an elongated tubular member serving as a sheath;
a temperature sensor for detecting the temperature of the medium;
a gas sensor for detecting the presence and/or determining the concentration of one or more target gases;
wherein the temperature sensor and the gas sensor are arranged in an interior of the elongated tubular member; and
an electronics unit having measurement- and operating circuitry, for operating the temperature sensor and the gas sensor.

The measurement- and operating circuitry serves for operating the temperature and the gas sensor, and for example, for processing and/or forwarding respective measurement signals generated by the temperature sensor and/or by the gas sensor.

The elongated tubular member is, for example, a measuring insert. The first and second temperature sensors are not limited to a particular type of sensing element. Thus, the invention includes, for example, the above-mentioned thermocouples and/or PTC or NTC thermistors or other temperature sensing elements which generate an, especially electrical, measuring signal in response to a change of temperature.

The advantage of the device is that the gas sensor being arranged together with the temperature sensor in the elongated tubular member, the presence of the gas is directly detectable in the vicinity of the temperature sensor.

In one embodiment of the apparatus, the gas sensor is embodied to detect the presence and/or determine the concentration of H2 as a first target gas and is especially embodied as a thin film sensor.

In one embodiment of the apparatus, the electronics unit and/or a device, which device is equipped to communicate with the electronics unit, is embodied to detect the presence and/or to determine the concentration of the one or more target gases within the elongated tubular member, based at least on a first measurement signal generated by the gas sensor.

The electronics unit and/or the device is, for example, embodied to generate a signal relating to an intrusion from the one or more target gas into the insside of the measuring insert. Said device is, for example, an, especially, mobile communication and/or control device. The device may be, for example, a smartphone, a tablet, data glasses or a mobile device specific to process automation, such as the FieldXpert sold by the Endress+Hauser Group. The device is equipped to communicate with the electronics unit via a communication link, for example embodied as a wired or a wireless communication link.

In one embodiment of the apparatus, the gas sensor comprises a first resistance temperature detector element, especially a first resistance temperature detector element having a negative temperature coefficient, and wherein the gas sensor is embodied such that the presence and/or concentration of the one or more target gases in a volume surrounding the gas sensor influences the temperature of first resistance temperature detector element.

In this case, the presence and/or concentration of the one or more target gases is determined indirectly, as, first, said presence and/or concentration in a volume surrounding the gas sensor, affects the temperature present in said volume, such that it can be picked up by the first resistance temperature detector element. The temperature of the first resistance temperature detector element is then picked up, for example, as an electrical signal generated by the first resistance temperature detector element, especially an electric current and/or voltage, serving as a first measurement signal generated by the gas sensor.

For this purpose, in a further embodiment of the above embodiment, the gas sensor comprises one or more catalytic layers in contact with the first resistance temperature detector element, which catalytic layers, when exposed to a critical amount of the one or more target gases, produce an exothermic or endothermic reaction, which endothermic or exothermic reaction results in a temperature change detectable with the first resistance temperature detector element.

Thus, for example, the presence of the one or more target gases produces a chemical reaction in a respective catalytic layer adapted for the respective target gas, which reaction results in said temperature change detectable with the first resistance temperature detector element temperature. Especially, the temperature change produced by the endothermic or exothermic reaction depends on the concentration of the one or more target gases.

In an embodiment of the apparatus, the electronics unit and/or the communication and/or control device is embodied to detect the presence and/or determine the concentration of the one or more target gases within the elongated tubular member, based at least on a first measurement signal generated by the gas sensor, as well as on a second measurement signal generated by the temperature sensor, Especially, the second measurement signal generated by the temperature sensor, or a temperature derived therefrom is considered in the determination of the presence and/or concentration of the one or more target gases.

For example, the second measurement signal or the temperature derived therefrom, is correlated with the first measurement signal of the gas sensor. In this way, cross-sensitivities of the gas sensor on the temperature of the medium are corrected for. It is thus possible to differentiate between a temperature change in the first resistance temperature detector element caused by a change of temperature of the medium and by the above catalytic reaction. This increases the accuracy of the determination of the presence and/or concentration of the one or more target gases.

For an even better correlation of the first measurement signal of the gas sensor and the second measurement signal of the temperatures, preferably, several of first measurement signals from the gas sensor and second measurement signals from the temperature sensor are considered. For example, several first and second measurement signals are picked up, wherein the several first and second measurement signals are respectively recorded at several measurement points in time, for example at consecutive measurement points in time. Eventually, a multitude, (for example at least ten) of first and second measurement signals, each recorded at different measurement points in time, are considered.

In an embodiment of the apparatus, the apparatus comprises two or more connection lines, especially three or more connection lines, which connection lines serve for contacting the temperature sensor and/or the gas sensor with the electronics unit.

For example, the connection lines are electrical connection lines, serving for conveying a measuring voltage and picking up an electric current as a first and/or second measuring signal.

The two or more connection lines serves for picking up the first measurement signal generated by the gas sensor as well as the second measurement signal generated by the temperature sensor. In this regard, each of the sensors could have their own, separate connections lines, or one or more connection lines could be shared. Additionally, the apparatus may comprise an interconnecting line, which serves for interconnecting gas sensor and the temperature sensor, or for interconnecting their connection lines.

In an embodiment of the apparatus, the gas sensor comprises a Palladium element, especially a thin film Palladium element.

In an embodiment of the apparatus, the temperature sensor comprises a second resistance temperature detector element, especially a second resistance temperature detector element having a positive temperature coefficient, preferably comprising Platinum, and/or wherein the temperature sensor comprises a thermoelement.

As mentioned earlier, the invention is not restricted to a specific type of temperature sensor and may especially also comprise a second resistance temperature detector element having a negative temperature coefficient.

In an embodiment of the apparatus, the elongated tubular member has a total length in a longitudinal direction, wherein the temperature sensor and the gas sensor are arranged in a sensing section of the elongated tubular member, which sensing section extends from the distal end region of the elongated tubular member towards a proximal end region of the elongated tubular member, and which sensing section has a sensing section length in the longitudinal direction of at most 1/3, especially at most 1/5, of the total length of the elongated tubular member. The sensing section length is thus defined by the total length in the longitudinal direction taken up by the temperature sensor and the gas sensor.

In an embodiment of the apparatus, the gas sensor and the temperature sensor are located directly adjacent to each other.

The gas sensor and the temperature sensor are, for example fastened together by a fastening means providing a material-locking and/or force-locking connection, such an adhesive connection using a glue, and/or a solder connection with a solder joint, and/or a rivet joint, etc. In this regard, the temperature sensor and the gas sensor are packaged together as one sensor element.

In an embodiment of the apparatus, the gas sensor occupies a volume smaller than 8 cm³, especially smaller than, 2 cm³, preferably smaller than 1 cm³. Thus, for such a small gas sensor, it can be easily assembled with the temperature sensor into the interior of the elongated tubular member 4, even when using an elongated tubular member with a small diameter, for example having a diameter of about 1 cm or bigger. Depending on the specific design, very small gas sensors occupying a volume smaller than 0,1 cm³ may be used for even smaller diameters of the elongated tubular member, for example a diameter of 0,3 cm.

In an embodiment of the apparatus, the apparatus comprises a gas-repelling layer, provided on one or more repelling surfaces of the apparatus, wherein the gas-repelling layer is equipped to impede a diffusion of the or more target gases.

For example, the elongated tubular member has a first outer surface, and a first repelling surface is provided in at least a region of the first outer surface.

In another example, when the apparatus comprises a protective tube which serves for accommodating the elongated tubular member and for insertion into the medium, the protective tube has a second outer surface, wherein a second repelling surface is provided on at least a region of the second outer surface.

The gas-repelling layer may be applied to the one or more repelling surfaces in form of a coating.

Regarding the method, the object underlying the invention is achieved by a method for operating an apparatus according to the invention, the method comprising:
- Determining a first measurement signal with the gas sensor;
- Determining a second measurement signal with the temperature sensor; and
- Detecting the presence and/or determine the concentration of the one or more target gases within the elongated tubular member, based at least on the first measurement signal generated by the gas sensor, as well as on a second measurement signal generated by the temperature sensor.

In an embodiment of the method, the method comprises:
Generating a message regarding the intrusion of the one or more target gases into the interior of the elongated tubular member, depending on the detected presence and/or determined concentration of the one or more target gas.

The message can be generated by the electronics unit of the apparatus, and/or the communication and/or control device.

In an embodiment of the method, the method comprises:
- Activation of a sealing mechanism, in case the presence of one or more target gases in the interior the elongated tubular member is detected,
   and wherein the sealing mechanism is equipped to impede a diffusion of the detected one or more target gases from the interior of the elongated tubular member into an environment of the apparatus.

The sealing mechanism which is equipped to impede a diffusion from the one or more target gases from the inside of the elongated tubular member into the environment in such case is, for example, similar compared to at least one embodiment described in the WO 2011/076481 A1. Therefore, with regards to the sealing mechanism, the present invention refers to WO 2011/076481A1 in its entirety.

Finally, the invention comprises also the use of an apparatus according to the invention, for determining and/or monitoring the temperature of a medium in a container especially a gas-tight closed and/or pressurized container, wherein the medium comprises H2.

The invention is explained in more detail with reference to the following figures, which are not to scale, where identical reference signs denote identical features. Where clarity so requires or it otherwise appears expedient, reference signs already mentioned may be omitted in subsequent figures.

The following are shown:
Fig. 1a and 1b show a schematic side view of an embodiment of the apparatus 100 according to the invention, and;
Fig. 2 shows a flowchart showing steps of the method according to the invention, in one embodiment of the invention.

A medium M is contained in a container 10, for example a vessel for containing the medium M or a tube for conduction the medium M in a process plant. The apparatus 100 is embodied to determine and/or monitor the temperature T of the medium contained in the container 10. For this purpose, the apparatus 100 protrudes over a process connection 13 into the container 10, with at least a section of an elongated tubular member 4 having a total length L, in a longitudinal direction. The elongated tubular member 4 acts as a sheath for a temperature sensor 1, for example a Pt100, or another type of the above-mentioned temperature sensors 1. The temperature sensor 1 is arranged close to a distal end region 41 of the elongated tubular member 4, at which distal end region 41 the elongated tubular member 4 is closed off. The apparatus 100 is inserted into the medium M, starting from its distal end region 41. A proximal end region 42 of the elongated tubular member 4 is opposite to the distal end region 41, in the longitudinal direction along the length L.

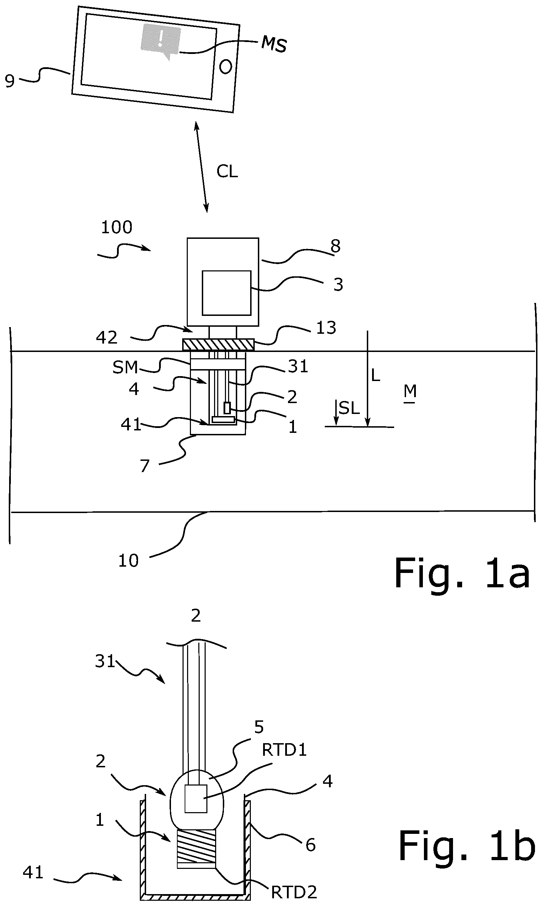

The apparatus 100 may additionally comprise, as shown in Fig. 1 but not essential for the invention, a protective tube 7. The protective tube 7 serves for accommodating the elongated tubular member and for insertion into the medium M whose temperature is determined and/or monitored. The apparatus 100 may comprise a transmitter housing 8 for housing an electronics unit 3. The transmitter housing 8 is attached to the elongated tubular member 4 at the proximal end region 42 of the elongated tubular member 4.

In particular, the gas-containing medium M is stored in a gas-tight, and especially pressurized container 10, for example containing H2 as a first gas. In this case, it is of importance to quickly and reliable detect the intrusion of gas out of the medium M into the elongated tubular member 4, which may be, for example, caused or reinforced by the aforementioned process known as hydrogen embrittlement.

Therefore, according to the invention, a gas sensor 2 is arranged in the interior of the elongated tubular member 4, in the vicinity of the temperature sensor 1. A sensing section having a sensing section length SL, which sensing section extends from the distal end region 41 and encompasses both the temperature sensor 1 and the gas sensor 2. The sensing section is thus in direct or indirect (when using a protective tube 7) contact with the medium M.

Both the gas sensor 2 and the temperature sensor 1 are contacted with the electronics unit 3 via connection lines 31. The invention is not limited to a specific number of connection lines; for example, as the temperature sensor 1 may be operated in a two-, three-, or four-wire circuit. Also, the gas sensor 2 may be contacted by two or more connection lines 31. The temperature sensor 1 and the gas sensor 2 may also share one or more of their respective connection lines 31 and the apparatus 100 may (not shown here) comprise an interconnecting line, which interconnecting line for example connects the connection lines 31 for contacting the temperature sensor 1 with the connection lines 31 for contacting the gas sensor 2. The connection lines 31 serve for transmitting a first measurement signal 21 of the gas sensor 2 and a second measurement signal 11 of the temperature sensor 1 to the electronics unit 3.

If applicable, the apparatus 100 may also comprises an electrically isolating filler (not shown here) into which the temperature sensor, the gas sensor 2, and at least part of the connection lines 31 are embedded.

The gas sensor 2 used in the invention for this purpose is, for example, embodied as the a thin film sensor, as for example described in the scientific article titled "Hydrogen Sensor Based on NTC Thermistor with Pt-Loaded WO3/SiO2 Coating" by Sun et al, published in Micromachines 2022, 13, 2219. 2022, 13, https://doi.org/10.3390/mi13122219. In this case, the gas sensor 1 is based on a first resistance temperature detector element RTD1, here a NTC chip thermistor, which first resistance temperature detector element RTD1 is embedded into an epoxide or ethoxyline resin matrix with least one catalytic layer 5. When being exposed to a critical amount of H2 as the at least one target gas, chemical reactions occur in the catalytic layers 5, owing to the existence of Pt. These chemical reactions are exothermic and therefore, by continuously generating heat, raise the temperature of the NTC thermistor until a thermal equilibrium is reached. This causes a temperature change in a volume surrounding the first resistance temperature detector element RTD1.

As a result, the gas concentration cg of H2 can be determined by monitoring a first measurement signal 2 of the gas sensor 2, picked up with the connection lines 31, for example a voltage across the NTC thermistor.

The invention is not limited to this type of gas sensor 2. For example, other types of thin film gas sensor 2 integrable into the interior of the elongated tubular member 4 comprise thin film sensors based on Palladium (Pd), wherein especially the adsorption of the target gas, especially H2, on the Palladium (Pd) thin film is determined. For example, this includes, but is not limited to,
- metal Oxide Semiconductor (MOS) thin film Pd sensors acting as capacitors or field effect transistors, wherein the adsorbed gas on the Palladium thin film forms a dipole at the metal oxide interface, changing the electrical characteristics of the capacitor or field effect transistor;
- thin film resistors, wherein a change in resistivity of a Pd in the presence of the gas is monitored to determine the presence and/or concentration of the target gas;
- pyroelectric sensors, wherein a thin film of Pd is deposited on the surface of a pyroelectric material, whose polarization is a function of temperature. Thus, when the target gas is adsorbed on the Pd surface, a heat of adsorption of the adsorption process produces a measurable potential difference, which is in turn indicative of the presence and/or concentration of the at least one target gas;
- piezoelectric sensors, wherein an oscillation frequency of a piezoelectric material is a function of its mass, which is determined by the amount of adsorption of H2 on a Pd coated piezoelectric surface, especially with a thin film Pd coating ;
- SAW (Surface Acoustic Wave) sensors, wherein adsorbed H2 on a Pd thin film perturbs surface acoustic waves on a piezoelectric substrate; and
- fiber optic sensors, wherein the adsorption of H2 on a Pd surface coating, especially a thin film Pd coating, on an optic fiber alters the optical property, (absorbance, reflectance, luminescence
   and/or scattering) of the fiber.

The advantage gas sensors based on Pd thin films is that they may be advantageously designed to be very small in size and can thus integrated together with the temperature sensor 1 into the interior of the elongated tubular member 4.

Consequently, the gas sensor 2 occupies a very small volume, f.e. smaller than 2 cm³ and the sensing section with the temperature sensor 1 and gas sensor 2 can be designed having a small sensing section length SL, for example of at most 1/3 of the total length of the elongated tubular member 4, even for small elongated tubular members 4.

The electronics unit 3 and/or a communication and/or control device 9 in communication with the electronics unit 3 is embodied to detect the presence and/or to determine the concentration of the one or more target gases within the tubular member 4, based at least on a first measurement signal 21 from the gas sensor 2, and, preferably, based on both the first measurement signal 21 and a second measurement signal 11 from the temperature sensor 1. The communication and/or control device 9 communicates over the communication link CL with the electronics unit 3, for example, a wireless communication link CL.

A more detailed view of the combination of the temperature sensor 1 and gas sensor 2 is shown in Fig. 1b. Here, the gas sensor 2 comprises the above-mentioned first resistance temperature detector element RTD1 with an NTC, embedded in a matrix with the catalytic layer 5. The temperature sensor 1 is also realized as an RTD and comprises a second resistance temperature detector element RTD2, for example a Pt-based RTD-element.

Preferably, the temperature sensor 1 and the gas sensor 2 are packaged together, for example by using a solder bond and/or an adhesive connection with a glue. When packaged together as one sensor element, the temperature sensor 1 and the gas sensor 2 are located directly adjacent to each other.

Furthermore, as shown in Fig. 1b, the elongated tubular member 4 comprises a gas-repelling layer 6, which hinders hydrogen permeation from the medium into the interior of the elongated tubular member 4, by impeding the diffusion of the one or more target gases. This gas-repelling layer 6 is provided on or more repelling surfaces of the apparatus.

For example, as shown in Fig. 1b the elongated tubular member 4 has a first outer surface, and a first repelling surface is provided in at least a region of the first outer surface When the apparatus 100 comprises a protective tube, as shown in Fig. 1a, similarly, a second repelling surface is provided on at least a region of a second outer surface of the protective tube 7. The gas-repelling layer is, for example, applied to the one or more repelling surfaces in form of a coating.

Preferably, especially when using a gas sensor 2 which detects the presence and/or determines the concentration of the target gas indirectly, via a temperature change caused by the at least one target gas, both a first measurement signal 21 of the gas sensor 2 and a second measurement signal 11 of the temperature sensor 1 is used.

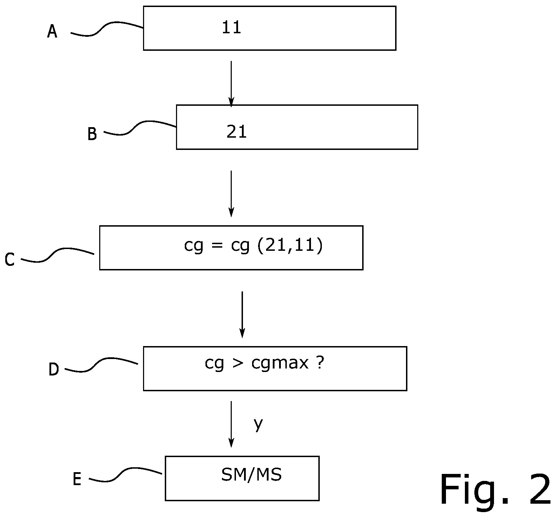

This is explained in more detail in conjunction with Fig. 2, showing the steps of the method in an embodiment of the method.

In step A) of Fig. 2, the second measurement signal 11 is picked up with the temperature sensor 1. In step B), the first measurement signal 21 is picked up with the gas sensor 2. In step C), the gas concentration cg of the one or more target gases is determined or its presence is detected.

Preferably, in step C), both the first measurement signal 21 as well as the second measurement signal 11 are considered. In this way, a temperature change picked up by the gas sensor, which is only caused by a change of the temperature T of the medium M, but not by the presence of the target gas within the interior elongated tubular member 4 is monitored and filtered. This increases the accuracy of the gas detection in the elongated tubular member 4.

Similarly (not shown in Fig. 2) the temperature experienced by the second resistance temperature detector element RTD2 of the temperature sensor 1 may be influenced by a change of temperature produced by the gas sensor 2, which is caused by the presence of the gas in the interior of the elongated tubular member 4. Therefore, also the temperature T determined by the electronics unit 3 may consider the first measurement signal 21. For example, the electronics unit 3 may consider especially a multitude, for example at least 5, measurement signals 12, 21 recurrently picked up at different points in time, both from the temperature sensor 1 as well as the gas sensor 2. By performing a temporal analysis of the two measurement signals 12,21 a temperature change caused by the temperature change of the medium and a temperature change caused by the presence of gas may be even better distinguished from one another.

As stated above, for this purpose, step A)-C), may also comprising considering several first measurement signals 21 and several second measurement signal 11, which are recorded at several measurement points in time. When correlating the several first and second measurement signals, especially in considering their chronological sequence, the accuracy of the determination of the gas concentration cg is thus increased.

Similarly, also for the determination of the temperature T, the several first and second measurement signals are correlated, especially considering their chronological sequence.

In step D), it is checked, if the gas concentration cg is greater than a maximal gas concentration cgmax. This maximal gas concentration cgmax is a value indicative of

Advantageously, see step E), the apparatus 100 comprises a sealing mechanism SM, which is especially activated, when the presence of a gas, especially with a maximal gas concentration cgmax, of the one or more target gases in the interior the elongated tubular member 4 is detected.

Finally, step E) comprises also the display of a message MS relating to the intrusion of gas into the interior of the elongated tubular member 4. This message is, for example, directly displayed on the apparatus 100, such as on a (Touch-)Display of the apparatus 100, f.e. arranged in the transmitter housing 8. Similarly, the message MS, as shown in Fig. 1, is displayed on a communication and/or control device 9 embodied to communicate the electronics unit 3 of the apparatus.

### Reference Signs and Symbols

- 100: apparatus
- 1: temperature sensor
- 11: measurement signal of 1
- 2: gas sensor
- 21: measurement signal of 2
- 3: electronics unit
- 31: connection lines
- 4: elongated tubular member
- 41: proximal end region
- 42: distal end region
- 5: catalytic layer
- 6: gas-repelling layer
- 7: protective tube
- 8: transmitter housing
- 9: communication and/or control device
- 10: container
- 13: process connection

- RTD1: first resistance temperature detector element
- RTD2: second resistance temperature detector element
- T: temperature
- M: medium
- L: length
- SL: sensing section length
- CL: communication link
- cg: gas concentration
- cgmax: maximal gas concentration

## Claims

1. Apparatus (100) for determining and/or monitoring the temperature (T) of a medium (M) in container (10), comprising:
an elongated tubular member (4) serving as a sheath;
a temperature sensor (1) for detecting the temperature of the medium;
a gas sensor (2) for detecting the presence and/or determining the concentration (cg) of one or more target gases;
wherein the temperature sensor (1) and the gas sensor (2) are arranged together in an interior of the elongated tubular member (4); and
an electronics unit (3) having measurement- and operating circuitry, for operating the temperature sensor (1) and the gas sensor (2).

2. Apparatus (100) according to Claim 1,
wherein the gas sensor (2) is embodied to detect the presence and/or determine the concentration (cg) of H2 as a first target gas and is especially embodied as a thin film sensor.

3. Apparatus (100) according to Claim 1 or 2,
wherein the electronics unit (3) and/or a communication and/or control device (9), which device is equipped to communicate with the electronics unit (3), is embodied to detect the presence and/or to determine the concentration (cg) of the one or more target gases within the elongated tubular member (4), based at least on a first measurement signal (21) generated by the gas sensor (2).

4. Apparatus (100) according to at least one of the preceding claims,
wherein the gas sensor (2) comprises a first resistance temperature detector element (RTD1), especially a first resistance temperature detector element (RTD1) having a negative temperature coefficient,
and wherein the gas sensor (2) is embodied such that the presence and/or concentration (cg) of the one or more target gases in a volume surrounding the gas sensor (2) influences the temperature of the first resistance temperature detector element (RTD1).

5. Apparatus (100) according to Claim 4, wherein the gas sensor (2) comprises one or more catalytic layers (5) in contact with the first resistance temperature detector element (RTD1),
which catalytic layers (5), when exposed to a critical amount of the one or more target gases, produce an exothermic or endothermic reaction, which endothermic or exothermic reaction results in a temperature change detectable with the first resistance temperature detector (RTD1) element.

6. Apparatus (100) according to at least one of the previous Claims,
wherein the electronics unit (3) and/or the communication and/or control device (9) is embodied to detect the presence and/or determine the concentration (cg) of the one or more target gases within the elongated tubular member (4), based at least on a first measurement signal generated (21) by the gas sensor (2), as well as on a second measurement signal generated (11) by the temperature sensor (1).

7. Apparatus (100) according to at least one of the preceding claims,
comprising two or more first connection lines (31) especially three or more connection lines (31), which connection lines (31) serve for contacting the temperature sensor (1) and/or the gas sensor (2) with the electronics unit (3).

8. Apparatus (100) according to at least one of the preceding claims, wherein the gas sensor (2) comprises a Palladium (Pd) element, especially a thin film Palladium element.

9. Apparatus (100) according to at least one of the preceding claims,
wherein the temperature sensor (1) comprises a second resistance temperature detector element (RTD2), especially a second resistance temperature detector element (RTD2) having a positive temperature coefficient, preferably comprising Platinum (Pt),
and/or
wherein the temperature sensor (2) comprises a thermoelement.

10. Apparatus (100) according to at least one of the preceding claims,
wherein the elongated tubular member (4) has a total length (L) in a longitudinal direction, and wherein the temperature sensor (1) and the gas sensor (2) are arranged in a sensing section of the elongated tubular member (4), which sensing section extends from a distal end region (41) of the elongated tubular member (4) towards a proximal end region (42) of the elongated tubular member (4), and which sensing section has a sensing section length (SL) in the longitudinal direction of at most 1/3, especially at most 1/5, of the total length (L) of the elongated tubular member (4).

11. Apparatus (100) according to at least one of the preceding claims,
wherein the gas sensor (2) and the temperature sensor (1) are located directly adjacent to each other.

12. Apparatus (100) according to at least one of the preceding claims,
wherein the gas sensor (2) occupies a volume smaller than 8 cm³, especially smaller than 2 cm³, preferably smaller than 1 cm³.

13. Apparatus (100) according to at least one of the preceding claims,
comprising a gas-repelling layer (6), provided on one or more repelling surfaces of the apparatus,
wherein the gas-repelling layer (6) is equipped to impede a diffusion of the one or more target gases.

14. Method for operating an apparatus (100) according to one of the previous Claims, the method comprising:
- Determining a first measurement signal (21) with the gas sensor (2);
- Determining a second measurement signal (11) with the temperature sensor (1); and
- Detecting the presence and/or determine the concentration (cg) of the one or more target gases within the elongated tubular member (4), based at least on the first measurement signal (21) generated by the gas sensor (2), as well as on a second measurement signal (11) generated by the temperature sensor (1).

15. Method according to Claim 14, comprising
- Generating a message (MS) regarding the intrusion of the one or more target gases into the interior of the elongated tubular member (4), depending on the detected presence and/or determined concentration (cg) of the one or more target gases.

16. Method according to Claim 15, comprising:
- Activation of a sealing mechanism (SM), in case the presence of one or more target gases in the interior the elongated tubular member (4) is detected, wherein the sealing mechanism (SM) is equipped to impede a diffusion of the detected one or more target gases from the interior of the elongated tubular (4) member into an environment of the apparatus (100).

17. Use of an apparatus (100) according to one of the Claims 1 to 13, for determining and/or monitoring the temperature (T) of a medium (M) in container (10), especially a gas-tight closed and/or pressurized container (10), wherein the medium comprises H2.
